# EUROPEAN PATENT APPLICATION

(11) **EP 2 133 313 A2**
(43) Date of publication of application: **16.12.2009**
(21) Application number: 09466005.7
(22) Date of filing: 26.05.2009
(51) Int. Cl.: C02F 11/00, C02F 1/00, C02F 103/00

(54) **Unit for decontamination of liquid and semi-solid waste**

(30) Priority: 28.05.2008 CZ 200819983 U
(71) Applicant: Nybro s.r.o., 61200 Brno (CZ)
(72) Inventor: Pink, Tomás, 61300 Brno (CZ)
(74) Representative: Kania, Frantisek

(57) **Abstract**

A unit for a decontamination of liquid and semi-solid waste according to the invention comprises a collecting piping (3) for liquid and semi-solid waste, the collecting piping (3) being provided with a bifurcated waste drain, wherein each branch of the bifurcated drain is provided with a shut-off valve (2ₐ) and wherein each of the branches leads into a decontamination tank (1, 4) in order to enable continuous collection of liquid and semi-solid waste by alternating a collecting function and a decontaminating function of the two decontamination tanks (1, 4) A vacuum pump (5) is connected to the waste drain or at least to one of the decontamination tanks (1, 4) for the purpose of draining off the liquid and semi-solid waste from the waste collecting piping (3).

## Description

### Field of the invention

The present invention relates to a unit for decontamination of liquid and semi-solid waste.

### Background of the invention

Hospitals, laboratories and other workplaces have often problems with disposal of hazardeous waste. Often liquid or semi-solid contaminated waste cannot be drained into the sewerage because the prevailing sewage treatment plants are not capable of decontaminating such kind of wastes and this could consequently lead to a contamination of the environment.

The most common unit for decontamination of such waste is a waste collector, the content of which is subsequently chemically decontaminated, e.g. by means of hypochlorites. Disadvantage of such chemical decontamination is that further chemicals are added to the sewerage water. Further disadvantage is a relatively low efficiency of chemical decontamination.

Another known method is thermal decontamination, whereas the content of the waste collector is heated up to the sterilizing temperature. However, during this process of decontamination, the contaminated waste is discontinuously drained away, which process is interrupted for the period of decontamination of the waste collector content. Draining the waste collector and furnishing the same for the next collection of a contaminated waste is possible only after decontamination of the content of the waste collector.

Both above mentioned methods are disadvantageous also in that only that part of waste which drains off to the waste collector is decontaminated, but no decontamination may be provided in the supply piping in which e.g. germs may multiply and communicate through this piping into all intakes of the supply piping, eventually they might also get in the atmosphere. Thereby a contamination of all locations from which the contaminated waste is collected may occur, due to a contaminant generated in one single point of waste collection only.

Further disadvantage is that the collection of contaminated waste is performed using the gravity so that there has to be superelevation which means in practice that it is not possible to process the contaminated waste in one floor only. The collection of the waste has to proceed at least one floor lower so it is necessary to process the contaminated waste in at least two floors. In case of a forced circulation is applied, i.e. the waste is urged by means of an overpressure, there is a risk of a waste leakage.

### Summary of the invention

It is the object of this invention to eliminate the mentioned disadvantages of prior art to a large extent by means of the below described unit for decontamination of liquid and semi-solid waste, whereas the principle of the invention is that the unit consists of a collecting piping for liquid and semi-soiid waste with a bifurcated drain whereas each branch of the bifurcated drain is provided with a shut-off valve and leads into a decontamination tank and a vacuum pump is connected to the drain for liquid and semi-solid waste or to at least one of the decontamination tanks for draining the liquid and semi-solid waste from the collecting piping, preferably through a HEPA filter. Preferably, the shut-off valves of the bifurcated drain are provided as a single three-way valve.

According to a preferred embodiment of the unit for decontamination of liquid and semi-solid waste each collection site for liquid and semi-solid waste comprises an inlet provided with a shut-off valve and a steam supply for a sterilization of the collecting piping for liquid and semi-solid waste is connected via the shut-off valve to the collecting piping.

According to another preferred embodiment of the unit for decontamination of liquid and semi-solid waste a steam supply for a sterilization of decontamination tanks and of the waste contained therein is connected to each of the decontamination tanks via a shut-off valve.

According to another preferred embodiment of the unit for decontamination of liquid and semi-solid waste the inlets in the waste collection sites are arranged in peripheries such as breeding cages, breeding chambers, collecting bowls, wash-basins, showers and sinks, which may be hermetically closed and which are made of a pressure and thermally resistant material so that their sterilization may be executed by means of a steam supply which is connected to the collecting piping.

According to another preferred embodiment of the unit for decontamination of liquid and semi-solid waste the collecting piping has a double-shell construction with an underpressure in the space between the shells, whereas preferably a pressure gauge is connected to the space between the shells.

It is also preferred that collecting piping in the unit for decontamination of liquid and semi-solid waste is provided with an outer shell constructed as a hermetically sealed collector or covered channel.

At least a part of the outer shell may be constructed from a transparent material for visual check of the condition of the collecting piping.

According to another preferred embodiment of the unit for decontamination of liquid and semi-solid waste the collecting piping along with the drain and with the decontamination tanks is located on the same level, whereas the ratio of the smoothness of the inner surface of the collecting piping to its inner diameter is selected so as to enable draining of liquid and semi-solid waste from those parts of the collecting piping, which elevate in the flow direction of contaminated liquid and semi-solid waste, without any residual liquid.

Finally, according to a further preferred embodiment of the unit for decontamination of liquid and semi-solid waste the drivers of the shut-off valves of the branches of the bifurcated drain, the drivers of the inlets in the waste collection sites and the drivers of the steam supplies for sterilization of the collecting piping are connected to computer a process control for controlling the process.

### Brief description of the drawings

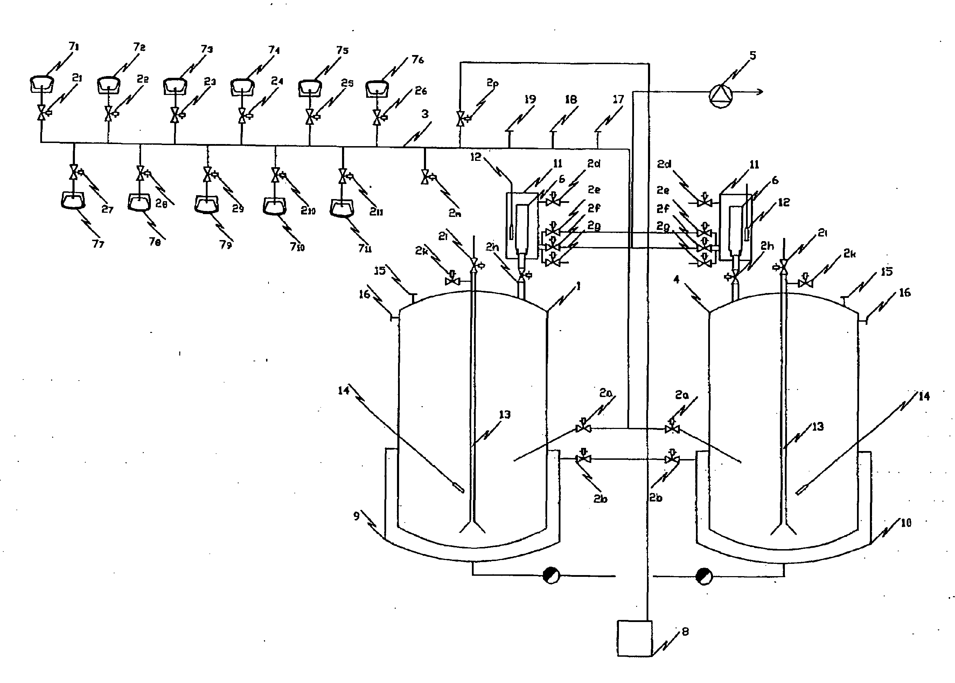

The invention will be explained in more detail referring to the enclosed Figure 1 which shows schematically an embodiment of the unit for decontamination of liquid and semi-solid waste according to the present invention.

### Detailed description of embodiments

Figure 1 depicts a first decontamination tank **1** connected via a shut-off valve **2** to a collecting piping **3** for liquid and semi-solid waste and a second decontamination thank **4** connected via another shut-off valve **2**ₐ to the collecting piping **3** as well. The vacuum plump **5** is connected via HEPA filters **6** to the first decontamination tank **1** as well as to the second decontamination tank **4.** HEPA filters **6** are located within heat insulated shieldings **11** provided with compressed air supply valves **2**_{d}, steam injection valves **2**ₑ, vacuum valves **2**_{f} and condensate drain valves **2**g. Thermometers **12** are inserted into the area between the isolated shieldings **11** and HEPA filters **6**. HEPA filters **6** are further connected to the decontamination tanks **1**, **4** via the shut-off valves **2**ₕ of the HEPA filters 6. The collecting piping **3** is further connected to the respective waste collection sites **7**₁ to **7**₁₁ via the respective shut-off valves **2**₁ to **2**₁₁. A steam supply **8** is connected to the collecting piping **3**.

There is delivery piping **13** centrally placed a both in the first decontamination tank **1** as well as in the second decontamination tank **4** and provided with a discharge valve **2**ᵢ and with a side valve **2**ₖ for a steam injection. The decontamination tanks **1**, **4** are further provided with thermometers **14** for measuring the inner temperature, with sensors **15** for measuring the internal pressure and with sensors **16** for measuring the level.

The collecting piping **3** is made of stainless steel and it has a double-shell construction. The space between the shells of the collecting piping **3** is connected to the vacuum pump **5** via the valve **2**ₘ. The collecting piping 3 is connected via the steam injection valve **2**ₚ to the steam supply **8** The collecting piping 3 is further provided with a pressure sensor **17**, with a temperature sensor **18** and with a pressure sensor **19** for the collector system.

Under operation of the unit for decontamination of liquid and semi-solid waste according to the present invention the shut-off valve **2**ₐ of the first decontamination tank **1** is open and the shut-off valve **2**ₐ of the second decontamination tank **4** is closed. The contaminated liquid waste is generated inside the respective waste collection sites **7**₁ to **7**₁₁ and it is drained through the shut-off valve **2**ₐ of the first decontamination tank **1** into the first decontamination tank **1** due to the underpressure inside the collecting piping **3**, which is caused by the drainage of the collecting piping **3** by means of the vacuum pump **5**. Hereby the HEPA filters **6** ensure that the contamination does not get into the atmosphere along with the exhausted air. During this process, a sterilization of the system may be performed anytime. It is only to be ensured that the system is sealed either by closing all shut-off valves **2**₁ to **2**₁₁ of the respective waste collection sites **7**₁ to **7**₁₁ or by closing the periphery and opening the shut-off valve **2**ₙ of the n-th waste collection site **7**ₙ arranged just in that particular periphery.

In this patent application, the term "periphery" means for example breeding cages, breeding chambers, collecting bowls, wash-basins, showers and sinks. Such units may be closed hermetically and they are made of pressure and thermally resistant material in order to facilitate their sterilization by means of steam supply connected to the waste collecting piping. Such breeding cages and breeding chambers may be constructed like stainless steel cylinders with sight-glasses e.g. from borosilicate glass wherein the air is supplied and wherefrom the air and further media are discharged through HEPA filters. In case of death of an experimental animal, the breeding cage or breeding chamber may be sterilized and after the sterilization the breeding cage or breeding chamber may be cleaned for further experiments.

After the system is sealed and those peripheries inside which are humans or experimental animals or other experimental materials not suitable for sterilization are eliminated from the system by closing the respective n-th shut-off valves **2**ₙ of the waste collection sites **7**ₙ, the hot steam is supplied from the steam supply 8 into the waste collecting piping **3**, penetrating into each open route, i.e. not only into the waste collecting piping **3**, but also into the first decontamination tank **1** and even through the open n-th shut-off valves **2**ₙ of the waste collection sites **7**ₙ into the selected peripheries. In this way a sterilization not only of the content of the first decontamination tank **1**, but of the complete waste collecting piping **3** and even of some peripheries is performed at the same time. Simultaneously the content of the decontamination tank **1** is sterilized by its heating by supply steam through the steam injection valve **2**_{b} into the double-shell **9** of the first decontamination tank **1**. The content of the first decontamination tank is agitated by a steam pulse injection through the side steam injection valve **2**ₖ into the delivery piping **13**, which is simultaneously sterilized by this steam. The whole process is generally controlled by the control computer, controlling all shut-off valves **2**ₙ and at the same time documenting all the processes in progress.

After sterilization it is possible to drain the already decontaminated content of the first decontamination tank **1** into the sewerage.

At least for the period of sterilization of the first decontamination tank **1** the liquid and semi-solid waste is collected in the second decontamination tank **4**. After draining off the decontaminated content of the first decontamination tank **1** into the sewerage it is possible to connect the first decontamination tank **1** to the waste collecting piping **3** by opening the respective waste supply valve **2**ₐ and to disconnect the second decontamination tank 4 from the waste collecting piping 3 by closing the respective waste supply valve **2**ₐ. Then the sterilization of the second decontamination tank **4** and of its content may be performed. In this manner the collection of liquid and semi-solid waste may be performed continuously, whereby the processes of waste collection and waste sterilization in each of the decontamination tanks **1**, **4** regularly alternate.

It is possible to sterilize only the first decontamination tank **1**, when the shut-off valve **2**ₐ of the first decontamination tank **1** is closed and the shut-off valve **2**_{b} for the steam injection into the double-shell **9** of the first decontamination tank **1** between the steam generator **8** and the first decontamination tank **1** is closed and the shut-off valve **2**ₐ of the second decontamination tank **4** is open, while the shut-off valve **2**_{b} for steam injection into the double-shell **10** of the second decontamination tank 4 between the steam generator **8** and the second decontamination tank **4** is closed. After the sterilization of the content of the first decontamination tank **1** the shut-off valve **2**_{b} between the steam generator **8** and the first decontamination tank **1** is closed, the already decontaminated liquid waste is drained off and the first decontamination tank **1** is ready for opening of its shut-off valve **2**ₐ after the shut-off valve **2**ₐ of the second decontamination tank **4** is closed for a sterilization of its content after opening the shut-off valve **2**_{b} between the steam generator **8** and the second decontamination tank **4**. In this manner it possible to collect the accumulated waste continuously without interrupting the collection of contaminated liquid and semi-solid waste and to perform its sterilization in one tank, whereas the collection is performed in the second tank.

### Utilization of the invention

The unit may be utilized in the workplaces which produce contaminated liquid waste such as hospital departments and medical or biological laboratories.

## Claims

1. A unit for a decontamination of liquid and semi-solid waste, **characterized in that** it comprises a collecting piping (3) for liquid and semi-solid waste, the collecting piping (3) being provided with a bifurcated waste drain, wherein each branch of the bifurcated drain is provided with a shut-off valve (2ₐ) and wherein each of the branches leads into a decontamination tank (1, 4) in order to enable continuous collection of liquid and semi-solid waste by alternating a collecting function and a decontaminating function of the two decontamination tanks (1, 4) and wherein a vacuum pump (5) is connected to the waste drain or at least to one of the decontamination tanks (1, 4) for the purpose of draining off the liquid and semi-solid waste from the waste collecting piping (3).

2. The unit for decontamination of liquid and semi-solid waste according to claim 1, **characterized in that** the shut-off valves (2ₐ) of the bifurcated waste drain are provided as a single three-way valve.

3. The unit for decontamination of liquid and semi-solid waste according to claim 1 or 2, **characterized in that** the vacuum pump (5) is connected to the waste drain or to at the least one decontamination tank (1, 4) via a HEPA filter (6).

4. The unit for decontamination of liquid and semi-solid waste according to any of claims 1 through 3, **characterized in that** each inlet into the waste collecting piping (3) inside a waste collection site (7ₙ) is provided with a shut-off valve (2ₙ) and that a steam supply (8) is connected via a shut-off valve (2ₚ) to the waste collecting piping (3) for a sterilization of the waste collecting piping (3).

5. The unit for decontamination of liquid and semi-solid waste according to any of claims 1 through 4, **characterized in that** the steam supply (8) is connected to each of the decontamination tanks (1, 4) via the shut-off valve (2ₑ) for a sterilization of the decontamination tanks (1, 4) and of the liquid and semi-solid waste contained therein.

6. The unit for decontamination of liquid and semi-solid waste according to claim 4 or 5, **characterized in that** the inlets inside the waste collection sites (7ₙ) are arranged in peripheries such as breeding cages, breeding chambers, collecting bowls, wash-basins, showers and sinks, which are hermetically closable and made of pressure resistant and thermally resistant material to enable their sterilization by means of the steam supply (8) connected to the waste collecting piping (3).

7. The unit for decontamination of liquid and semi-solid waste according to claim 6, **characterized in that** all unit inlets and drains are hermetically sealable in order to enable a testing of overpressure and underpressure in the whole system.

8. The unit for decontamination of liquid and semi-solid waste according to any of claims 1 through 7, **characterized in that** the waste collecting piping (3) has double-shell construction with underpressure in the space between the shells.

9. The unit for decontamination of liquid and semi-solid waste according to claim 8, **characterized in that** the external shell of the waste collecting piping (3) is a hermetically sealed collector or a shielded channel.

10. The unit for decontamination of liquid and semi-solid waste according to claim 8 or 9, **characterized in that** at least a part of the outer shell of the waste collecting piping (3) is constructed from a transparent material for a visual check of the condition of the waste collecting piping (3).

11. The unit for decontamination of liquid and semi-solid waste according to claim 8, **characterized in that** a pressure sensor (19) is connected to the space between the shells for the purpose of testing the integrity of that space under low pressure.

12. The unit for decontamination of liquid and semi-solid waste according to any of claims 1 through 9, **characterized in that** the waste collecting piping (3) along with the drain and with the decontamination tanks (1, 4) is located on the same level, wherein the ratio of the smoothness of the inner surface of the waste collecting piping (3) to its inner diameter is selected such as to enable draining off liquid and semi-solid waste from those parts of the waste collecting piping (3), which elevate in the flow direction of contaminated liquid and semi-solid waste, so that no liquid remains therein.

13. The unit for decontamination of liquid and semi-solid waste according to any of claims 1 through 9, **characterized in that** the controllers of the shut-off valves (2ₐ) of the bifurcated drain branches, of the inlets inside the waste collection sites (7ₙ) and of the steam supplies (8) for the sterilization of the waste collecting piping (3) are connected to control outputs of a process control computer.
